# EUROPÄISCHE PATENTSCHRIFT

(11) **EP 4 118 444 B1**
(45) Veröffentlichungstag und Bekanntmachung des Hinweises auf die Patenterteilung: **09.04.2025**
(21) Anmeldenummer: 21717995.1
(22) Anmeldetag: 04.03.2021
(51) Int. Cl.: G01R 33/341, G01R 33/3415, G01R 33/36, H01Q 5/314, H01Q 7/00

(54) **DOPPELTRESONANTE SPULE SOWIE ARRAY VON DOPPELTRESONANTEN SPULEN UND DEREN VERWENDUNG**
DOUBLE-RESONANT COIL, ARRAY OF DOUBLE-RESONANT COILS, AND USE THEREOF
BOBINE À DOUBLE RÉSONANCE ET RÉSEAU DE BOBINES À DOUBLE RÉSONANCE ET LEUR UTILISATION

(30) Priorität: 13.03.2020 DE 102020001654; 21.01.2021 DE 102021000282
(43) Veröffentlichungstag der Anmeldung: 18.01.2023
(73) Patentinhaber: Forschungszentrum Jülich GmbH, 52425 Jülich (DE)
(72) Erfinder: HONG, Suk Min, 52146 Würselen (DE); FELDER, Jörg, 52428 Jülich (DE); SHAH, Nadim Joni, 52428 Jülich (DE); CHOI, Chang-Hoon, 52428 Jülich (DE)
(86) Internationale Anmeldenummer: PCT/DE2021/000043
(87) Internationale Veröffentlichungsnummer: WO 2021/180259

(56) Entgegenhaltungen:
- US-A- 5 365 173
- US-A1- 2009 160 442
- US-B2- 9 864 032
- SEUNGHOON HA ET AL: "A PIN diode controlled dual-tuned MRI RF coil and phased array for multi nuclear imaging; PIN diode controlled dual-tuned MRI RF coil", PHYSICS IN MEDICINE AND BIOLOGY, INSTITUTE OF PHYSICS PUBLISHING, BRISTOL GB, vol. 55, no. 9, 7 May 2010 (2010-05-07), pages 2589 - 2600, XP020171895, ISSN: 0031-9155
- INTERNATIONAL SOCIETY FOR MAGNETIC RESONANCE IN MEDICINE, ISMRM, 2030 ADDISON STREET, 7TH FLOOR, BERKELEY, CA 94704 USA, no. 4400, 1 June 2018 (2018-06-01), XP040703608

## Beschreibung

Die Erfindung betrifft eine doppeltresonante Spule sowie ein Array von doppeltresonanten Spulen und deren Verwendung.

Die Erfindung betrifft eine Spulenanordnung zur Verwendung als Sende- und/oder Empfangsspule in einem MRT System oder einem MR/NMR-Spektroskopie-System, die aus einer oder mehreren physikalischen Leiterstrukturen besteht, auf denen sich jeweils zwei verschiedene Stromverteilungen anregen lassen. Jede einzelne Leiterstruktur weist so zwei verschiedene Resonanzfrequenzen auf. Außerdem betrifft die Erfindung ein MRT-System oder MR/NMR-Spektroskopie-System mit einer solchen Spulenanordnung. Darüber hinaus betrifft die Erfindung die Verwendung einer solchen Spulenanordnung sowie ein Array von doppeltresonanten Spulen und deren Verwendung.

Die Magnetresonanztomographie, kurz MRT, ist ein in der medizinischen Diagnostik eingesetztes Bildgebungsverfahren mittels dem Struktur, Funktion und Metabolismus von Geweben insbesondere in Form von Schnittbildern dargestellt werden. In der MR/NMR Spektroskopie werden Verschiebungen der kernmagnetischen Resonanzfrequenzen aufgrund lokaler Variationen des zugrundeliegenden statischen Magnetfelds als Spektren oder Bilder dargestellt. Aufgrund der Ähnlichkeit beider Verfahren werden MRT-Systeme und MR/NMR-Spektroskopie-Systeme im Folgenden einheitlich als MR-Systeme bezeichnet. Die Aufnahmen mit beiden Systemen werden einheitlich als MR-Bildgebung bezeichnet.

Neben der überwiegend eingesetzten MR-Bildgebung basierend auf Protonen (Wasserstoffkerne, ¹H) können auch Kernresonanzen anderer Kerne mit einem von Null verschiedenen Kernspinn aufgenommen werden. Diese anderen Kerne werden als X-Kerne zusammengefasst und umfassen z. B. metabolisch aktive Kerne wie Natrium (²³Na) und Phosphor (³¹P). Sie weisen aufgrund des kernspezifischen Wertes der gyromagnetischen Konstante eine andere Resonanzfrequenz als der Wasserstoffkern auf. Aufgrund der zumeist deutlich geringeren Konzentration von X-Kernen in biologischem Gewebe und der kleineren Resonanzfrequenz ist das Empfangssignal bei X-Kern MR-Bildgebung geringer als bei MR-Bildgebung mittel Wasserstoffkernen. Bei Ultrahochfeld MRT mit einer statischen Feldstärke von ≥7T, kurz UHF-MRT, kann bei X-Kern Messungen trotzdem ein vergleichsweise hohes Signal-zu-Rauschverhältnis gegenüber Messungen bei üblichen klinischen Feldstärken von 1.5T bis 3T, erzielt werden. Trotzdem werden X-Kern Messungen üblicherweise in Kombination mit MR-Bildgebung mittels Protonen durchgeführt. Dies erlaubt zum einen die Überlagerung von Informationen aus der X-Kern Messung auf anatomische Bilder, welche mittels MR-Bildgebung des Wasserstoffkerns aufgenommen wurden. Zum anderen werden Übersichtsaufnahmen, sogenannte Scout-Bilder, und Justagen, z.B. B₀ Shimming, mittels ¹H Messungen durchgeführt.

Die Bestrebungen gehen deshalb dahin, Spulenanordnungen zu generieren, die Kernresonanzen von Protonen und X-Kerne anregen und/oder detektieren können. Konkret muss die Spulenanordnung also in der Lage sein, hochfrequente elektromagnetische Felder auf der¹H Resonanzfrequenz und auf mindestens einer Resonanzfrequenz eines X-Kernes abstrahlen und/oder detektieren zu können. Eine doppeltresonante Spulenanordnung kann demnach elektromagnetische Felder auf der Protonenresonanzfrequenz und der Resonanzfrequenz einer ausgewählten Sorte von X-Kernen abstrahlen und/oder anregen.

Eine weitere Bestrebung, insbesondere für das Empfangen des von Kernresonanzen ausgesendeten elektromagnetischen Wechselfeldes, ist die Empfangsspulen möglichst nah am Untersuchungsvolumen zu positionieren und eine hohe Anzahl von Einzelspulen zu verwenden. Beides maximiert das Signal-zu-Rauschverhältnis des Empfangssignals. Insbesondere ist es von Interesse diese Elemente vorteilhaft in hochkanalige Empfangsarrays, wie sie beispielsweise in [1] beschrieben sind, in der Hochfeld-MRT zu verwenden. Da Spulenanordnungen meist für ein bestimmtes Untersuchungsvolumen optimiert sind, z. B. für den menschlichen Kopf in der Neurobildgebung, führt die Erhöhung der Anzahl von Spulenelementen, die auch als Kanalanzahl bezeichnet wird, zwangsläufig zu Einzelelementen mit geringeren geometrischen Abmessungen. Dies wiederum erschwert die Abstimmung von herkömmlichen Oberflächenspulen, insbesondere für hohe Resonanzfrequenzen wie sie in der UHF-MRT auftreten. Zum Beispiel beträgt die Abstimmkapazität einer kreisrunden Oberflächenspule mit einem freien Durchmesser von 85 mm 1.4 pF bei 7 T, 0.79 pF bei 9.4 T, 0.5 pF bei 11. 7T und 0.35 pF bei 14 T [2].

Zahlreiche Methoden zum Bau von doppelt oder mehrfach abgestimmten MRT Spulenanordnungen, wie sie für kombinierte ¹H/X-Kern Messungen erforderlich sind, sind bekannt. Alle diese Methoden streben die Entkopplung von Protonen und X-Kern Resonanzen an. Sie können folgendermaßen kategorisiert werden:
So wird die Entkopplung mittels Sperrkreisen, z.B. doppelt abgestimmte Birdcage-Resonatoren mit passiven Sperrkreisen meist auf alternierenden Beine beschrieben [3-5].

Weiterhin ist die Entkopplung mittels PIN Dioden aus [6] bekannt. Dies kann z.B. bei Verwendung von zwei unabhängige RF Spulen, die abwechselnd verstimmt werden, eingesetzt werden.

Die Veröffentlichungen [7-8] zeigen Geometrische Entkopplung, z.B. in Form von einer "Butterfly Spule".

Weiterhin sind modifizierte Resonator Strukturen bekannt, z.B. Birdcage Resonatoren mit zusätzlichen Endringen [9].

Dipol- und Monopolantennen können in doppelt abgestimmten Systemen in Kombination mit Oberflächenspulen verwendet werden. Werden sie in der Mitte der Oberflächenspule angeordnet, so resultiert, aufgrund der Magnetfelder beider Leiteranordnungen, ein verschwindender, gekoppelter magnetischer Fluss [10-12].

Aus der Antennentechnik sind gefaltete Dipolantennen bekannt. Diese können an den Dipolenden durch eine ohmsche Last verbunden und so über einen weiten Frequenzbereich betrieben werden [13], ebenfalls beschrieben in [14].

Hochkanalige Empfangs-Spulenanordnungen für die Neurobildgebung in der MRT sind in [15-16] beschrieben.

Monoresonante, gebogene Dipolantennen mit einer unsymmetrischen Stromverteilung entlang des Leiters (auch als "Loopole" bezeichnet) sind in [17] beschrieben. Sie werden als vorteilhaft bei einer einzelnen Resonanzfrequenz in der Hochfeld-MRT beschrieben, da sie sowohl partiell konservative und nicht-konservative Magnetfelder detektieren können.

Eine Möglichkeit Elementkopplung mithilfe eines Filternetzwerks zwischen den Einzelelementen zu realisieren, wird in [18] beschrieben. Die Entkopplung mittels Filternetzwerken ist jedoch nur bei einer Frequenz möglich.

Die Schrift [19] offenbart eine Verkürzung von Dipolantennen mittels Induktivitäten.

Wie bereits zuvor beschrieben lassen sich kleine Oberflächenspulen bei hoher Kanalzahl und hohen Feldstärken praktisch nicht abstimmen. Zwar kann die Kapazität durch die Reihenschaltung von zwei oder mehreren Kondensatoren auf einen realisierbaren Wert für jeden einzelnen Kondensator gebracht werden. Jedoch führt die Reihenschaltung zu höheren elektrischen Verlusten aufgrund einer aus der Reihenschaltung resultierenden niedrigeren Güte des Resonanzkreises und damit zu einer Verschlechterung des Signal-zu-Rausch Verhältnisses der MRT Bilder. Ebenfalls problematisch ist die Toleranz der aus einer Serienschaltung von Einzelkondensatoren resultierenden Gesamtkapazität. Diese kann den Wert der zuvor genannten Gesamtkapazität überschreiten.

Doppeltresonante Spulen können mittels Sperrkreisen realisiert werden. Dabei entkoppelt der Sperrkreis zusätzliche Kondensatoren von der Spule bei der ¹H Frequenz. Die zusätzlichen Kondensatoren sind also nur während der Resonanz der Spule bei der X-Kern Frequenz aktiv. Dies wiederum führt dazu, dass auch in doppeltresonanten Spulen die Abstimmung auf der ¹H Frequenz - wie zuvor beschrieben - schwierig bleibt.

Alternative Methoden zur Generierung doppeltresonanter Oberflächenspulen, sind aufgrund des zusätzlich benötigen Bauraums in hochkanaligen Spulenarrays oft nicht oder nur mit sehr hohem Aufwand realisierbar. Der zusätzliche Aufwand beinhaltet unter anderem, zusätzliche Leitungen zur Versorgung von PIN Dioden und einen größeren Platzbedarf der Einzelelemente durch zusätzliche Leiterbahnen nahe den Spulenelementen.

Alternative Antennenanordnungen, wie in [17] beschrieben, sind bisher nicht in doppeltresonanter Ausführung beschrieben.

Eine Möglichkeit Elementkopplung mithilfe eines Filternetzwerks zwischen den Einzelelementen zur realisieren, wird in [18] beschrieben. Die Entkopplung mittels Filternetzwerken ist jedoch nur bei einer Frequenz möglich.

Die Schrift [19] offenbart eine aus Leiterschleifen bestehende Spule, welche in Segmente unterteilt ist, die jedoch eine Varaktor-Diode umfasst, welche für nur den Empfang von Signalen geeignet ist.

In der Veröffentlichung [20] wird eine gerade Dipolantenne dargestellt, welche durch die Verwendung von parallelen Schwingkreisen ("Sperrkreisen"), elektrisch verkürzt werden kann. Dies ermöglicht den Betrieb der Anordnung als doppelt resonanten Dipol - in der MRT z. B. für kombinierte 1 H/X-Kern Messungen.

Es ist daher Aufgabe der Erfindung eine doppeltresonante Spule zur Verfügung zu stellen, die als Element für einen mehrkanaligen Aufbau in einer Spulenanordnung verwendet werden kann. Jedes Element soll neben der ¹H-Resonanz eine weitere Resonanz auf der Resonanzfrequenz eines ausgewählten X-Kerns aufweisen. Die doppeltresonante Spule soll bei einer hohen Anzahl an Kanälen und kleinen physikalischen Abmessungen auch bei hohen Feldstärken (UHF-MRT) gut abstimmbar sein. Die zusätzlichen ohmschen Verluste, durch das Einfügen von Bauteilen gegenüber monofrequenten Spulenausführungen, sollen sowohl auf der ¹H Resonanzfrequenz, als auch auf der X-Kern Resonanzfrequenz gering bleiben, so dass das Signal-zu-Rauschverhältnis der MRT-Bilder ähnlich demjenigen bei Verwendung monofrequenter Spulen ist. Der Aufbau soll mittels Kondensatoren mit handelsüblichen Toleranzwerten möglich sein. Die doppeltresonante Spule oder eine aus Spulen dieser Art aufgebaute Anordnung soll ohne zusätzlichen Platzbedarf für Zuleitung von PIN Dioden und eine Vielzahl von passiven Bauteilen auskommen. Protonen und X-Kern Resonanzen sollen hinreichend entkoppelt sein. Es soll eine Entkopplung der Einzelspulen des Spulenarrays untereinander bei mindestens zwei Frequenzen ermöglicht werden.

Ausgehend vom Oberbegriff des Anspruchs 1 und der nebengeordneten Ansprüche wird die Aufgabe erfindungsgemäß gelöst mit den im kennzeichnenden Teil angegebenen Merkmalen.

Mit der erfindungsgemäßen doppeltresonanten Spule ist es nunmehr möglich, neben ¹H auch andere X-Kerne zu vermessen, wobei sie als Element für einen mehrkanaligen Aufbau verwendet werden kann. Die doppeltresonante Spule ist bei hoher Kanalzahl, insbesondere bei hohen Feldstärken, die üblicherweise bei ≥ 7T liegen, gut abstimmbar. Es entstehen keine zusätzlichen elektrischen Verluste, beispielsweise auf durch die Serienschaltung verlustbehafteter Kondensatoren. Ein hohes Signal-zu-Rausch Verhältnis von MRT Bildern und Spektren bleibt auf beiden Resonanzfrequenzen gewährleistet. Hohe Toleranzen, hervorgerufen durch die Serienschaltung mehrere Einzelkondensatoren, werden vermieden. Die doppeltresonante Spule oder ein aus Spulen dieser Art gebautes Spulenarray ist ohne größeren Platzbedarf der Einzelelemente, hervorgerufen durch zusätzliche Leiterbahnen und/oder Bauteile, realisierbar. Protonen- sowie X-Kern Resonanzen auf den Einzelspulen sowie zwischen den Elementen einer aus diesen Elementen aufgebauten Spulenanordnung können entkoppelt werden.

Vorteilhafte Weiterbildungen der Erfindung sind in den Unteransprüchen angegeben.

Im Folgenden wird die erfindungsgemäße doppeltresonante Spule in ihrer allgemeinen Form dargestellt, ohne dass dies einschränkend auszulegen ist.

Der Erfindung liegt die Überlegung zugrunde, eine physikalische Struktur sowohl als Dipolantenne, nämlich mit dipolartiger Stromverteilung bei der ¹H Resonanzfrequenz, als auch als herkömmliche Oberflächenspule, mit homogener Stromverteilung bei der X-Kern Resonanzfrequenz, zu verwenden. Gleichzeitig soll das so entstehende Spulenelement für den Einsatz in hochkanaligen Spulenarrays verwendbar sein. Dies erfordert insbesondere, dass es geometrisch kompakt gestaltet werden kann. Werden die Enden eines gefalteten Dipols mit einem Kondensator - also nicht ohmsch - verbunden, erinnert die so entstandene Struktur an eine herkömmliche Oberflächenspule. Mittels eines Verbindungselements, welches von einem elektrisch leitenden in einen elektrisch trennenden Zustand überführt werden kann, kann die Geometrie frequenzselektiv in einem Dipolmode, wobei das Verbindungselement für die Protonenfrequenz sperrt, als auch in einem Oberflächenspulen-Mode mit homogener Stromverteilung, wobei das Verbindungselement eine andere Impedanz als bei ¹H für die X-Kern Frequenz aufweist, betrieben werden. Da für die Protonenfrequenz keine kapazitive Abstimmung notwendig ist, die Abstimmung im Dipolmode erfolgt über die Leiterlänge eventuell auch unter Zuhilfenahme von Methoden zur Verkürzung oder Verlängerung von Dipolen, können die Nachteile des Stands der Technik insbesondere der Bedarf von Kondensatoren mit kleinen, nicht realisierbaren Kapazitätswerten, vermieden werden. Elektromagnetische Simulationen der Anordnung haben gezeigt, dass die hier beschriebene Methode mindestens bis zu Feldstärken von 14 T für Antennenarrays mit 32 Kanälen für Aufnahmen des menschlichen Kopfes realisierbar ist.

Erfindungsgemäß wird eine doppeltresonante Spule, aus einer geschlossenen Leiterschleife, die in mindestens zwei Segmente unterteilt ist, zur Verfügung gestellt. Die Segmente sind über jeweils ein Verbindungselement verbunden bzw. überbrückt, wobei das Verbindungselement von einem elektrisch leitenden in einen elektrisch isolierenden Zustand überführt werden kann. Die über die Verbindungselemente verbundenen Segmente bilden die Leiterschleife. Das Verbindungselement ist ein Mittel, welches von einem elektrisch leitenden in einen elektrisch isolierenden Zustand überführt und wird im Folgenden der Einfachheit halber als Verbindungselement bezeichnet.

Die Leiterschleife kann aus dafür üblichen Materialien bestehen, also beispielsweise aus Metallbändern, Drähten oder gedruckt auf einem Trägermaterial.

Beide Verbindungselemente können beispielsweise ein Sperrkreis, eine PIN-Diode oder eine gleichwirkende funktionellen Einheit sein, wobei der Sperrkreis eine bevorzugte Ausführungsform ist, da für seinen Betrieb keine aktive Ansteuerung benötigt wird.

Die Geometrie der Leiterschleife ist frei wählbar und kann so gewählt werden, dass sie bei der Anwendung an einem Körperteil, beispielsweise an einem Kopf gut positioniert werden kann. Sie kann kreisförmig, oval, rund oder eckig gebogen, mehreckig, beispielsweise viereckig in langgezogener Geometrie, quadratisch, fünfeckig, sechseckig usw. ausgebildet sein.

Beide Verbindungselemente sind innerhalb der Leiterschleife vorzugsweise so angeordnet, dass sie sich an Positionen befinden, die die Länge der Leiterschleife hälftig teilen, da hierdurch eine Mitteneinspeisung des Dipolmodes bei der ¹H Frequenz ermöglicht wird.

Einem ersten Verbindungselement ist eine Induktivität oder ein Kondensator in Serie geschaltet, welche zur Anpassung und/oder zur Abstimmung der Anordnung auf die Resonanzfrequenz der X-Kerne verwendet werden kann.

Die Leiterschleife verfügt über Punkte zur Einspeisung elektrischer Energie. Diese sind beidseitig des ersten Verbindungselements angeordnet. Der Abstand zu dem ersten Verbindungselement ist variabel.

Der Anordnung aus dem ersten Verbindungselement und in Serie geschaltetem Kondensator oder Induktivität ist eine weitere Induktivität oder ein weiterer Kondensator parallel geschaltet. Der parallel geschaltete Kondensator oder die parallel geschaltete Induktivität hat zur Folge, dass die Spule auf die Resonanzfrequenz des ¹H Kerns angepasst werden kann.

Die anderen Enden der beiden Segmente der Leiterschleife sind durch ein zweites Verbindungelement verbunden, welches bei der ¹H Frequenz sperrt und damit bei dieser Frequenz eine dipolartige Stromverteilung erzwingt.

Diesem Verbindungselement kann eine Induktivität oder ein Kondensator in Serie geschaltet sein. Das hat den Vorteil, dass eine Modifikation der Stromverteilung in der Leiterschleife für die X-Kern Resonanz vorgenommen werden kann.

Entlang der Leiterschleife können weitere Kondensatoren und/oder Induktivitäten bzw. mindestens ein weiterer Kondensator oder mindestens eine weitere Induktivität angeordnet sein, welche zur Verkürzung oder Verlängerung der elektrischen Länge der Leiteranordnung auf der ¹H und/oder X-Kern Resonanz verwendet werden können.

Vorteilhaft bestimmt sich die Anzahl der Kondensatoren und/oder Induktivitäten nach der effektiven elektrischen Länge der Leiterschleife. Hierbei werden ausreichend viele Kondensatoren und/oder Induktivitäten entlang der Leiterschleife angeordnet, um eine homogene Stromverteilung auf der X-Kernfrequenz zu erzielen.

Beispielsweise kann ein Kondensator und/oder eine Induktivität in der Leiterschleife eingefügt sein, der vorteilhaft mit dem zweiten Verbindungselement in Serie geschaltet ist.

Das hat zur Folge, dass eine homogene Stromverteilung gewährleistet ist. Eine andere Position ist aber auch möglich.

Die Kondensatoren in der Leiterschleife haben vorzugsweise Kapazitäten ≥ 1pF da anderenfalls das Problem besteht, das kleine Werte, die unter 1 pF liegen, die eng toleriert werden müssen und entsprechend teuer sind verwendet werden müssen, was vorteilhaft ausgeschlossen werden soll. Grundsätzlich ist die Höhe der Kapazitäten nach oberen Werten nicht beschränkt und eher durch praktische Gegebenheiten bedingt. Die Kapazitäten der Kondensatoren sind zweckmäßig durch den Bereich kommerziell verfügbarer konzentrierter, nicht magnetischer Kondensatoren beschränkt. Dieser Bereich liegt üblicherweise zwischen 0.5 pF und 1 nF.

Die Leiterschleife kann weiterhin durch mindestens einen Kondensator oder mindestens eine Induktivität unterbrochen sein. Es können aber auch 2, 3, 4, 5, 6, 7., 8, 9 bis beispielsweise 20 Kondensatoren und/oder bzw. Induktivitäten in die Leiterbahn integriert sein. Die Anzahl ist grundsätzlich nach oberen Werten offen und lediglich durch praktische Gegebenheiten begrenzt. Dadurch kann eine Verkürzung oder Verlängerung der elektrischen Länge der Leiterschleife erreicht werden. Die Position der Kondensatoren und/oder Induktivitäten ist frei wählbar.

Sollen diese, zur Verkürzung oder Verlängerung der elektrischen Länge der Leiterschleife verwendeten Induktivitäten oder Kondensatoren nur bei einer Resonanzfrequenz der doppeltresonanten Spule wirken, so können diese jeweils auch über ein Verbindungselement in Serie angeschlossen werden, welches von einem elektrisch leitenden in einen elektrisch sperrenden Zustand überführt werden kann. In diesen Fall ist die Parallelschaltung eines weiteren Kondensators oder einer weiteren Induktivität erforderlich, die für beide Frequenzen aktiv ist, damit die Leiterschleife der Spule nicht für eine Frequenz unterbrochen wird.

Die doppeltresonante Spule kann in mindestens zwei Exemplaren zu einem Array angeordnet werden, welches es ermöglicht verschiedene Körperregionen einer MRT Messung zugänglich zu machen. Es kann eine Anzahl von beispielsweise 2 bis 128 Spulen zu einem Array zusammen verwendet werden, die idealerweise um das zu vermessende Organ herum angeordnet sind. Eine hohe Anzahl von einzelnen doppeltresonanten Spulen formt ein hochkanaliges Array und führt zu MRT Bildern mit einem höheren Signal-zu-Rauschverhältnis. Zudem werden durch eine hohe Anzahl von Empfangskanälen die Eigenschaften des Spulenarrays bezüglich Methoden der parallelen Bildgebung verbessert.

Zur Entkopplung der Spulenelemente bei der X-Kernresonanz können aufgrund der homogenen Stromverteilung bekannte Entkopplungsmechanismen als Mittel zum Entkoppeln von Spulen verwendet werden. Für die Entkopplung der Protonenresonanz wird erfindungsgemäß ein Filternetzwerk zwischen den Eingängen benachbarter Spulen eingefügt. Damit das Filternetzwerk die Anpassung der Spule bei der X-Kernresonanz nicht verändert, muss jedes Filterelement mit einem Verbindungselement versehen werden, welches von einem elektrisch leitenden in einen elektrisch sperrenden Zustand überführt werden kann.

Die einzelnen Spulen sollen vorzugsweise eine kleine Baugröße aufweisen um möglichst viele Elemente auf einer gegebenen Oberfläche anordnen zu können. Die Durchmesser werden nach unten durch die gewünschte Eindringtiefe des elektromagnetischen Feldes in das Untersuchungsvolumen und nach oben durch die minimale gewünschte Anzahl von Elementen des Arrays begrenzt. Übliche Durchmesser im Bereich der Neurobildgebung liegen beispielsweise zwischen 30 cm und 10 mm.

Mit der erfindungsgemäßen doppeltresonanten Spule kann auch bei kleinen Abmessungen der Spulen und hohen Magnetfeldern von ≥ 7T eine verlässliche Abstimmung vorgenommen werden. Die erfindungsmäße Spule oder das erfindungsgemäße Array von Spulen kann bei hohen Feldstärken von beispielsweise 7 T bis 21 T für die Gewinnung sehr guter MRT Bilder herangezogen werden. Die Feldstärken, bei denen die Spulen eingesetzt werden können, sind grundsätzlich nach oberen Werten offen und lediglich durch die praktische Realisierbarkeit von Magneten mit entsprechenden Feldstärken begrenzt.

Die doppeltresonante Spule oder das Array von doppeltresonanten Spulen kann für ¹H Kerne und X-Kerne verwendet werden. Als X-Kerne können alle Kerne verwendet werden, die einen von null verschiedenen Kernspin aufweisen und daher in der MRT Bildgebung einsetzbar sind, beispielsweise aber nicht beschränkend ²H, ⁷Li, ¹³C, ¹⁷O, ¹⁹F,²³Na, ³¹P, ³⁵Cl und ³⁹K.

In einer vorteilhaften Ausgestaltung verfügt die erfindungsgemäße doppeltresonante Spule über zwei weitere Verbindungselemente, welche die doppeltresonante Spule während des Sendens mit einer anderen zusätzlichen Sendespule sperren. Diese Verbindungselemente verhindern einen Stromfluss während des Sendens auf den beiden Resonanzfrequenzen, nämlich derjenigen des ¹H und derjenigen des X-Kerns. Beide Verbindungselemente können PIN-Dioden oder antiparallele PN-Dioden sein. Die Ausgestaltung der Verbindungselemente ist dem Fachmann bekannt.

In einer vorteilhaften Ausgestaltung der erfindungsgemäßen doppeltresonanten Spule können die Punkte zur Einspeisung elektrischer Energie für beide Resonanzfrequenzen verwendet werden. Hierfür ist bei der Verwendung von schmalbandigen Vorverstärkern, wie sie in der MRT üblicherweise zur Optimierung des SNR verwendet werden, eine frequenzelektive Aufteilung der Empfangssignale notwendig. Die Methoden hierfür sind dem Fachmann bekannt.

Gegenstand der Erfindung ist auch ein Array von den erfindungsgemäßen doppeltresonanten Spulen. In dem Array sind die doppeltresonanten Spulen so angeordnet, dass sie um ein Körperteil, insbesondere um einen Kopf, herum angeordnet sind.

Die doppeltresonanten Spulen oder ein Array davon kann in NMR/MR-Spektroskopie bzw. der MR-Tomographie verwendet werden.

Die Figuren zeigen die erfindungsgemäße doppeltresonante Spule und deren Ausgestaltungen in schematischer Form.

Es zeigt:
Fig. 1: eine einfache Ausführungsform der erfindungsgemäßen Spule
Fig. 2: eine erfindungsgemäße Spule
Fig. 3: eine Spule nach Figur 1 mit Anschluss der Empfangskette.
Fig. 4: Messkurven a und b
Fig. 5: ³¹P Spektren

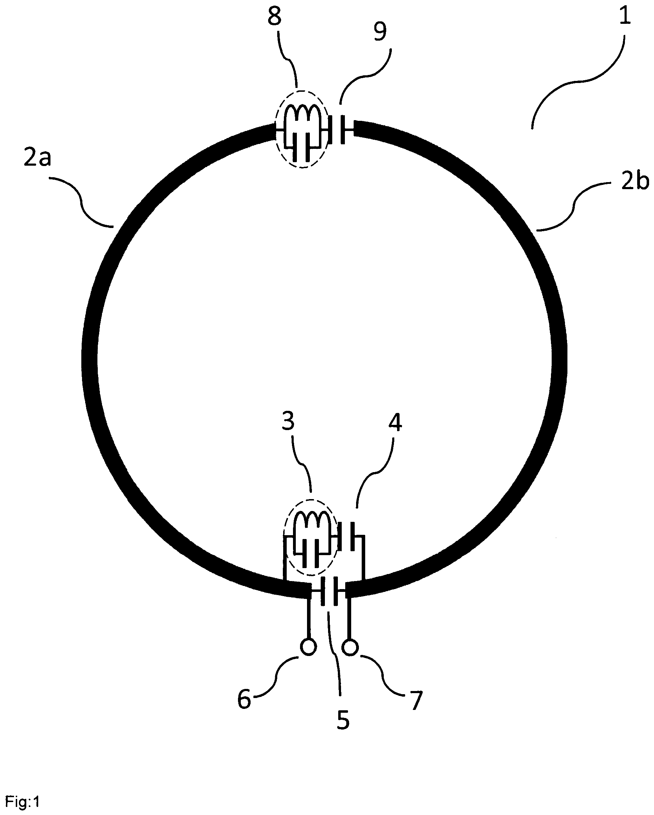

Figur 1 zeigt eine einfache Ausführungsform der doppeltresonanten Spule mit einer Leiterschleife 1 aus zwei Segmenten 2a, 2b. Sie verfügt über ein als Sperrkreis 3 ausgebildetes erstes Verbindungselement, dem ein Kondensator 4 in Serie geschaltet ist. Dem mit dem Kondensator 4 in Serie geschaltete Sperrkreis 3 ist ein Kondensator 5 parallel geschaltet. Weiterhin sind beidseitig des Kondensators 4 mit in Serie geschalteten Sperrkreis 3, Eingänge 6, 7 zur Einspeisung elektrischer Energie angeordnet. Die beiden Enden der Segmente 2a, 2b sind über ein zweites, als Schwingkreis 8 ausgebildetes Verbindungselement verbunden. Dem zweiten Verbindungselement ist ein weiterer Kondensator 9 in Serie geschaltet.

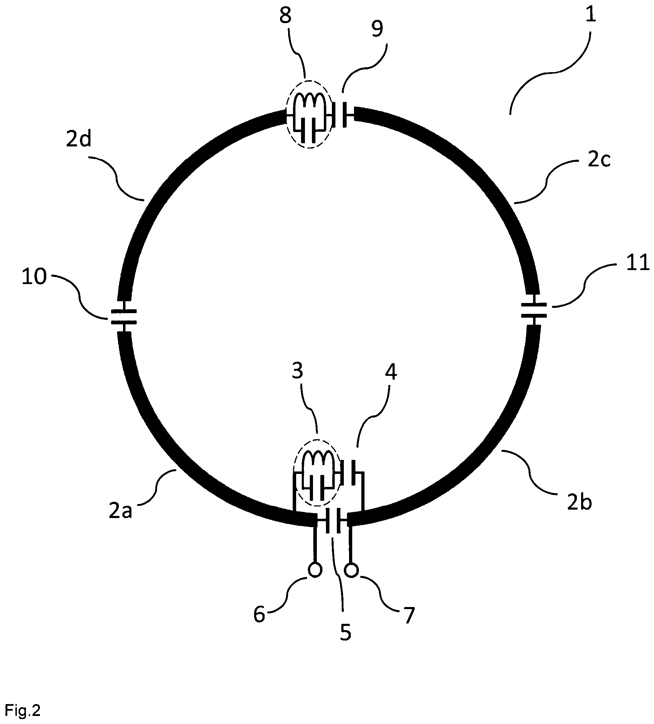

Figur 2 zeigt eine Ausführungsform der erfindungsgemäßen doppeltresonanten Spule. In ihr haben gleiche Vorrichtungsmerkmale dieselben Bezugszeichen. In dieser Ausführungsform finden sich an Stelle der beiden Segmente 2a, 2b vier Segmente 2a, 2b, 2c, 2d von denen jeweils 2 durch Kondensatoren 10, 11 verbunden sind.

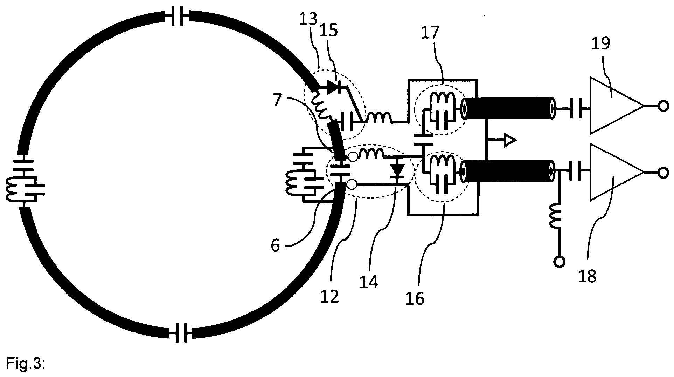

Figur 3 zeigt eine Möglichkeit des Anschlusses der Spule aus Figur 1 an eine Empfangskette für die beiden Resonanzfrequenzen. In ihr haben gleiche Vorrichtungsmerkmale dieselben Bezugszeichen. Weiterhin sind in Figur 3 zwei zusätzliche Verbindungselemente ausgeführt als Verstimmkreise 12, 13 angeordnet, die bei der Verwendung der Spule nach Figur 1 als Empfangselement die Verstimmung auf beiden Resonanzfrequenzen während des Sendepulses erlauben. Die Verbindungselemente, ausgeführt als Verstimmkreise 12 und 13, sind mittels PIN Dioden 14 und 15 schaltbar. Die Frequenzweiche zur Aufteilung des für beiden Resonanzfrequenzen an den Einspeisepunkten 6, 7 abgegriffenen Empfangssignals wird aus den beiden Resonanzkreisen 16 und 17 gebildete. Diese führen das Empfangssignal den schmalbandigen Vorverstärkern 18 und 19 zu.

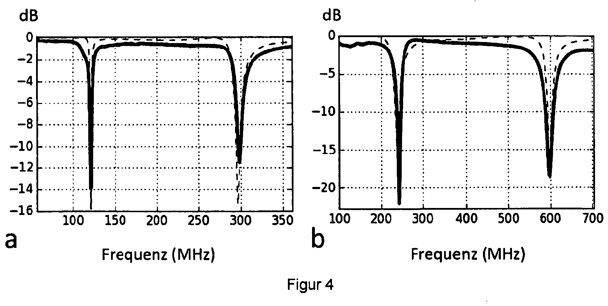

Figur 4 zeigt zwei Messkurven a und b, welche den Eingangsreflektionsfaktor einer erfindungsgemäßen doppeltresonanten Spule darstellen. Abbildung a zeigt den Eingangsreflektionsfaktor für eine Spule die bei einer statischen Feldstärke von 7 T für Protonen und Phosphor (³¹P) verwendet werden kann. Abbildung b zeigt eine Ausführung für 14 T. In beiden Abbildungen sind die Messwerte an einem Prototypen als durchgezogene Linie dargestellt und diejenigen aus einer 3D-Feldsimulation als gestrichelte Kurve.

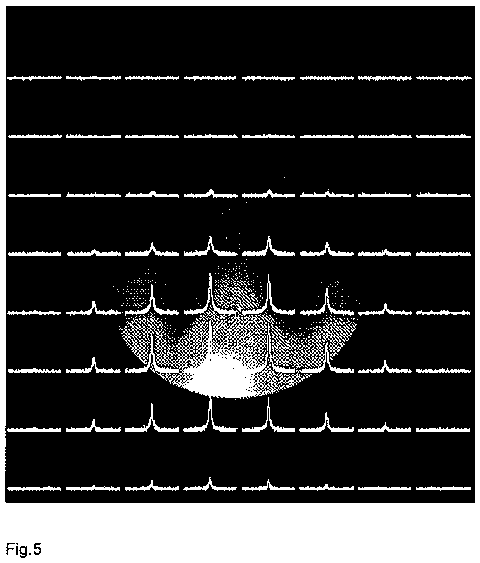

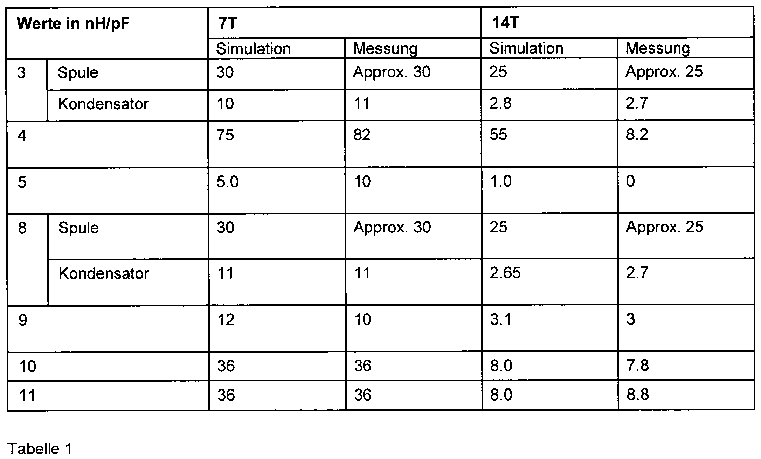

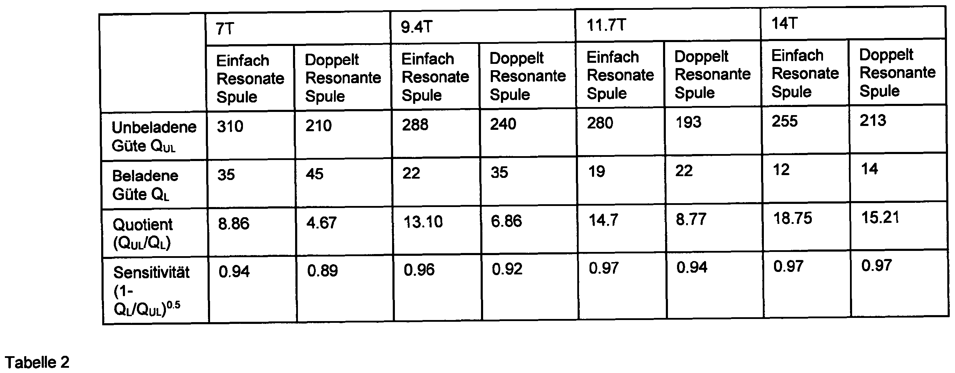

Figur 5 zeigt ein mit einer erfindungsgemäßen Spule aufgenommenes Protonenscoutbild eines MRT Phantoms. Überlagert sind lokalisierte ³¹P Spektren, welche ebenfalls mit der erfindungsgemäßen Spule aufgenommen sind.

Weiterhin werden folgende Tabellen dargestellt:
Tabelle 1: Werte der konzentrierten Bauteile von erfindungsgemäßen Prototypen
Tabelle 2: Sensitivität des doppeltresonanten Spulenelements verglichen mit derjenigen eines gleichartigen monofrequenten Elements auf der X-Kern Resonanz

### Beispiel:

Für ein hochkanaliges Empfangsarray kann z.B. ein Elementdurchmesser von ca. 85 mm geeignet sein. Ausgehend von dieser Dimension kann ein kreisförmig gebogener Dipol erstellt werden, dessen Resonanz aufgrund der Leiterlänge ca. 490 MHz entspricht. Figur 1 (ohne Verbindungselemente 3, 8 und ohne Kondensatoren 4, 9) zeigt die Anordnung des Dipols, in den zusätzlich konzentrierte Bauteile, hier Kondensatoren, zur Abstimmung und Frequenzanpassung eingebracht wurden. Die erforderlichen konzentrierten Bauteile können je nach ¹H Resonanzfrequenz auch Spulen sein oder durch analoge, nicht konzentrierte Elemente nachgebildet werden.

Für den Betrieb als X-Kern Oberflächenspule muss der Sperrkreis 8 auf die Protonenfrequenz abgestimmt werden. Die X-Kern Resonanz wird dann über 9 eingestellt.

Es kann vorteilhaft sein, einen weiteren Sperrkreis 3 sowie einen weiteren Kondensator 4 in der Schaltung einzufügen. Dies vereinfacht die Abstimmung und Anpassung der X-Kern Resonanz, wenn 3 wiederum bei der Protonenfrequenz eine hohe Impedanz (Resonanz) aufweist.

Ein Simulationsmodell und der Prototyp der einzelnen Spulenanordnung wurden verwendet, um den Eingangsreflektionsfaktor der Anordnung zu bestimmen. Die gemessenen und simulierten Resonanzen (¹H/³¹P) sind in Figur 4 dargestellt - für 7 T in Figur 4a und für 14 T in Figur 4b. Die für beide Frequenzen verwendeten Induktivitäts- und Kapazitätswerte sind in Tabelle 1 wiedergegeben. Tabelle 2 zeigt, dass die X-Kern Spule gegenüber einer monofrequenten Anordnung nur wenig an Sensitivität verliert, so dass die doppelt abgestimmte Anordnung nur geringe SNR Einbußen gegenüber einer nur auf die X-Kern Frequenz abgestimmten Spule aufweist.

Eine Validierung der Anordnung im MRT ist in Figur 5 gezeigt. Dort ist neben dem Protonenbild, aufgenommen mit einem einzelnen Spulenelement, auch das damit gewonnene lokalisierte Phosphor Spektrum dargestellt.

Eine leicht modifizierte Version der erfindungsgemäßen Spule ist in Figur 3 dargestellt. Hier werden die ¹H und ³¹P Empfangskanäle über einen gemeinsamen Abgriff an dem Spulenleiter abgeführt. Diese Anordnung ermöglicht vorteilhaft die Verstimmung des Empfangselements mittels PIN Dioden sowohl während der Anregung bei der ¹H als auch bei der ³¹P Resonanzfrequenz.

### Literatur

[1] B. C. Rowland et al., 'Whole brain P MRSI at 7T with a dual-tuned receive array', Magnetic Resonance in Medicine, vol. 0, no. 0.
[2] G. C. Wiggins, C. Triantafyllou, A. Potthast, A. Reykowski, M. Nittka, and L. L. Wald, '32-channel 3 Tesla receive-only phased-array head coil with soccer-ball element geometry', Magn Reson Med, vol. 56, no. 1, pp. 216-223, Jul. 2006.
[3] Shen GX, Wu J f., Boada FE, Thulborn KR. Experimentally verified, theoretical design of dual-tuned, low-pass birdcage radiofrequency resonators for magnetic resonance imaging and magnetic resonance spectroscopy of human brain at 3.0 Tesla. Magn. Reson. Med. 1999;41:268-275. doi: 10.1002/(SICI)1522-2594(199902)41:2<268::AID-MRM9>3.0.CO;2-G.
[4] Meyerspeer M, Roig ES, Gruetter R, Magill AW. An improved trap design for decoupling multinuclear RF coils. Magn. Reson. Med. 2013:n/a-n/a. doi:
   10.1002/mrm.24931.
[5] Dabirzadeh A, McDougall MP. Trap design for insertable second-nuclei radiofrequency coils for magnetic resonance imaging and spectroscopy. Concepts Magn. Reson. Part B Magn. Reson. Eng. 2009;35B:121-132. doi: 10.1002/cmr.b.20139.
[6] Ha S, Hamamura MJ, Nalcioglu O, Muftuler LT. A PIN diode controlled dual-tuned MRI RF coil and phased array for multi nuclear imaging. Phys. Med. Biol. 2010;55:2589-2600. doi: 10.1088/0031-9155/55/9/011.
[7] Bottomley PA, Hardy CJ, Roemer PB, Mueller OM. Proton-decoupled, overhauserenhanced, spatially localized carbon-13 spectroscopy in humans. Magn. Reson. Med. 1989;12:348-363. doi: 10.1002/mrm.1910120307.
[8] Adriany G, Gruetter R. A half-volume coil for efficient proton decoupling in humans at 4 tesla. J. Magn. Reson. San Diego Calif 1997 1997;125:178-184. doi: 10.1006/jmre.1997.1113.
[9] Potter W m., Wang L, McCully K k., Zhao Q. Evaluation of a new 1H/31P dual-tuned birdcage coil for 31P spectroscopy. Concepts Magn. Reson. Part B Magn. Reson. Eng. 2013;43:90-99. doi: 10.1002/cmr.b.21239.
[10] Shajan G, Mirkes C, Buckenmaier K, Hoffmann J, Pohmann R, Scheffler K. Threelayered radio frequency coil arrangement for sodium MRI of the human brain at 9.4 Tesla. Magn. Reson. Med. 2015:n/a-n/a. doi: 10.1002/mrm.25666.
[11] Yan X, Wei L, Xue R, Zhang X. Hybrid Monopole/Loop Coil Array for Human Head MR Imaging at 7 T. Appl. Magn. Reson. 2015:1-10. doi: 10.1007/s00723-015-0656-5.
[12] Yan X, Xue R, Zhang X. A monopole/loop dual-tuned RF coil for ultrahigh field MRI. Quant. Imaging Med. Surg. 2014;4:225-231.
[13] Elmer R. Bush, Broad bandwidth folded dipole antenna, US4423423A
[14] An Experimental All-Band Nondirectional Transmitting Antenna by Gil L. Countryman, W1RBK, (W3HH), QST, June 1949, S. 54.
[15] Wiggins, G.C., Polimeni, J.R., Potthast, A., Schmitt, M., Alagappan, V. and Wald, L.L. (2009), 96-Channel receive-only head coil for 3 Tesla: Design optimization and evaluation. Magn. Reson. Med., 62: 754-762. doi:10.1002/mrm.22028
[16] Keil, B., Blau, J.N., Biber, S., Hoecht, P., Tountcheva, V., Setsompop, K., Triantafyllou, C. and Wald, L.L. (2013), A 64-channel 3T array coil for accelerated brain MRI. Magn Reson Med, 70: 248-258. doi:10.1002/mrm.24427
[17] K. Lakshmanan, M. Cloos, and G. C. Wiggins, 'Circular dipole and surface coil loop structures and methods for using the same', US20150137815A1, 21-May-2015.
[18] I. R. O. Connell and R. S. Menon, "Shape Optimization of an Electric Dipole Array for 7 Tesla Neuroimaging," in IEEE Transactions on Medical Imaging, vol. 38, no. 9, pp. 2177-2187, Sept. 2019. doi: 10.1109/TMI.2019.2906507
[19] US2229865 "Radio antenna system
[19] US9864032 B2 Magnetic resonance imaging system.
[20] Suk-Min Hong, Chang-Hoon Choi, Jörg Felder, and N. Jon Shahlz, "Design and simulation of dual-band dipole antenna for 1H/31P at 9.4T MRI" Proc. Intl. Soc. Mag. Reson. Med. 26 (2018) page 4400

## Patentansprüche

1. Doppeltresonante Spule, zur Verwendung als Sende- und/oder Empfangsspule in einem Magnetresonanztomographie-System oder einem Magnetresonanz/NMR-Spektroskopie-System, wobei die Spule aus einer geschlossenen Leiterschleife besteht, wobei sie in mindestens zwei Segmente (2a, 2b) unterteilt ist, die jeweils über ein Verbindungselement verbunden sind, welches von einem elektrisch leitenden in einen elektrisch isolierenden Zustand überführt werden kann, wobei die Spule **dadurch gekennzeichnet ist, dass** dem Verbindungselement eine Induktivität oder ein Kondensator (9) in Serie geschaltet ist, was die Anregung der Leiterschleife (1) in einem Dipolmode bei der ¹H Frequenz und mit einer homogenen Stromverteilung bei einer zweiten Frequenz eines anderen als X-Kern bezeichneten Kern ermöglicht, der einen von null verschiedenen Spin aufweist.

2. Doppeltresonante Spule nach vorhergehendem Anspruch,
**dadurch gekennzeichnet,**
**dass** das Verbindungelement ein Sperrkreis (3) oder eine PIN-Diode (14, 15) ist.

3. Doppeltresonante Spule nach Anspruch 1 oder 2,
**dadurch gekennzeichnet,**
**dass** das Verbindungselement die Leiterschleife (1) hälftig teilt.

4. Doppeltresonante Spule nach einem der Ansprüche 1 bis 3,
**dadurch gekennzeichnet,**
**dass** dem Verbindungselement ein Kondensator (4) oder eine Induktivität parallel geschaltet ist.

5. Doppeltresonante Spule nach einem der Ansprüche 1 bis 4,
**dadurch gekennzeichnet,**
**dass** sie über eine Einspeisung für elektrische Energie verfügt.

6. Doppeltresonante Spule nach Anspruch 5,
**dadurch gekennzeichnet,**
**dass** die Spule ein zweites Verbindungselement aufweist und die Einspeisung für elektrische Energie beidseitig des Verbindungselements angeschlossen ist.

7. Doppeltresonante Spule nach Anspruch 6,
**dadurch gekennzeichnet,**
**dass** an dem zweiten Verbindungselement ein Kondensator (4) oder eine Induktivität in Serie geschaltet ist.

8. Doppeltresonante Spule nach einem der Ansprüche 1 bis 7,
**dadurch gekennzeichnet,**
**dass** entlang der Leiterschleife (1) mindestens ein weiterer Kondensator und/oder mindestens eine weitere Induktivität angeordnet ist, welche zur Verkürzung oder Verlängerung der elektrischen Länge der Leiteranordnung auf der ¹H und/oder X-Kern Resonanz verwendet werden kann.

9. Doppeltresonante Spule nach einem der Ansprüche 1 bis 8,
**dadurch gekennzeichnet,**
**dass** die Kondensatoren Kapazitäten ≥ 1pF bis 1000pF aufweisen.

10. Doppeltresonante Spule nach einem der Ansprüche 1 bis 9,
**dadurch gekennzeichnet,**
**dass** sie über zwei weitere Verbindungselemente verfügt, welche die doppeltresonante Spule während des Sendens mit einer anderen Sendespule sperren.

11. Doppeltresonante Spule nach vorhergehendem Anspruch,
**dadurch gekennzeichnet,**
**dass** mehrere Spulen zu einem Array zusammengeschaltet sind.

12. Array von doppeltresonanten Spulen nach einem der Ansprüche 1 bis 11.

13. Array nach Anspruch 12,
**dadurch gekennzeichnet,**
**dass** es Mittel zur Entkopplung von einzelnen doppeltresonanten Spulen besitzt.

14. Array nach einem der Ansprüche 12 oder 13,
**dadurch gekennzeichnet,**
**dass** es eine Anzahl von 2 bis 128 Spulen besitzt.

15. Array nach einem der Ansprüche 12 bis 14,
**dadurch gekennzeichnet,**
**dass** die Spulen so angeordnet sind, dass sie einen Kopf wenigstens teilweise umschließen.

16. Verwendung einer Spule nach einem der Ansprüche 1 bis 11 oder eines Arrays nach einem der Ansprüche 12 bis 15 bei der Magnetresonanz-Spektroskopie oder Magnetresonanztomographie.

## Claims

1. A double-resonant coil for use as a transmitting and/or receiving coil in a magnetic resonance imaging system or a magnetic resonance/NMR spectroscopy system, wherein the coil consists of a closed conductor loop, wherein it is subdivided into at least two segments (2a, 2b), each of which is connected via a connecting element which can be converted from an electrically conductive to an electrically insulating state, the coil being **characterized in that** an inductor or a capacitor (9) is connected in series to the connecting element which enables the excitation of the conductor loop (1) in a dipole mode at the ¹H frequency and with a homogeneous current distribution at a second frequency of another nucleus referred to as an X-nucleus, which has a spin other than zero.

2. The double-resonant coil according to the preceding claim, **characterized in that** the connecting element is a blocking circuit (3) or a PIN diode (14, 15).

3. The double-resonant coil according to claim 1 or 2, **characterized in that** the connecting element divides the conductor loop (1) in half.

4. The double-resonant coil according to any one of claims 1 to 3, **characterized in that** a capacitor (4) or an inductor is connected in parallel to the connecting element.

5. The double-resonant coil according to any one of claims 1 to 4, **characterized in that** it has an infeed for electric power.

6. The double-resonant coil according to claim 5, **characterized in that** the coil has a second connecting element, and the infeed for electric power is connected on both sides of the connecting element.

7. The double-resonant coil according to claim 6, **characterized in that** a capacitor (4) or an inductor is connected in series to the second connecting element.

8. The double-resonant coil according to any one of claims 1 to 7, **characterized in that** at least one additional capacitor and/or at least one additional inductor is arranged along the conductor loop (1) which can be used to shorten or lengthen the electrical length of the conductor arrangement on the ¹H and/or X-core resonance.

9. The double-resonant coil according to any one of claims 1 to 8, **characterized in that** the capacitors have capacitances of from ≥ 1 pF to 1000 pF.

10. The double-resonant coil according to any one of claims 1 to 9, **characterized in that** it has two additional connecting elements which block the double-resonant coil during transmission with another transmitting coil.

11. The double-resonant coil according to the preceding claim, **characterized in that** a plurality of coils are connected together to form an array.

12. An array of double-resonant coils according to any one of claims 1 to 11.

13. The array according to claim 12, **characterized in that** it has means for decoupling individual double-resonant coils.

14. The array according to any one of claims 12 or 13, **characterized in that** it has a number of from 2 to 128 coils.

15. The array according to any one of claims 12 to 14, **characterized in that** the coils are arranged such that they at least partially enclose a head.

16. A use of a coil according to any one of claims 1 to 11 or of an array according to any one of claims 12 to 15 in magnetic resonance spectroscopy or magnetic resonance tomography.

## Revendications

1. Bobine à double résonnance à utiliser comme bobine d'émission et/ou de réception dans un système de tomodensitométrie par résonnance magnétique ou dans un système de spectroscopie par résonnance magnétique / RMN, dans laquelle la bobine est constituée d'une boucle conductrice fermée, dans laquelle elle est subdivisée en au moins deux segments (2a, 2b), qui sont reliés respectivement par un élément de liaison, lequel peut passer d'un état conducteur de l'électricité à un état isolant électriquement, dans laquelle la bobine est **caractérisée en ce qu'**une inductance ou un condensateur (9) est monté en série avec l'élément de liaison,
ce qui rend possible une excitation de la boucle (1) conductrice dans un mode dipolaire à la fréquence ¹H et, avec une répartition homogène du courant, à une deuxième fréquence d'un autre noyau, désigné comme noyau X, qui a un spin différent de zéro.

2. Bobine à double résonnance suivant la revendication précédente,
**caractérisée**
**en ce que** l'élément de liaison est un circuit (3) bouchon ou une diode (14, 15) PIN.

3. Bobine à double résonnance suivant la revendication 1 ou 2, **caractérisée**
**en ce que** l'élément de liaison divise la boucle (1) conductrice par moitié.

4. Bobine à double résonnance suivant l'une des revendications 1 à 3,
**caractérisée**
**en ce qu'**un condensateur (4) ou une inductance est monté en parallèle avec l'élément de liaison.

5. Bobine à double résonnance suivant l'une des revendications 1 à 4,
**caractérisée**
**en ce qu'**elle dispose d'une alimentation en énergie électrique.

6. Bobine à double résonnance suivant la revendication 5,
**caractérisée**
**en ce que** la bobine a un deuxième élément de liaison et l'alimentation en énergie électrique est raccordée des deux côtés de l'élément de liaison.

7. Bobine à double résonnance suivant la revendication 6,
**caractérisée**
**en ce qu'**un condensateur (4) ou une inductance est monté en série avec le deuxième élément de liaison.

8. Bobine à double résonnance suivant l'une des revendications 1 à 7,
**caractérisée**
**en ce que**, le long de la boucle (1) conductrice, est monté au moins un autre condensateur et/ou au moins une autre inductance, qui peuvent être utilisés pour raccourcir ou allonger la longueur électrique de l'agencement conducteur à la résonnance ¹H et/ou à noyau X.

9. Bobine à double résonnance suivant l'une des revendications 1 à 8,
**caractérisée**
**en ce que** les condensateurs ont des capacités ≥ 1 pF jusqu'à 1000 pF.

10. Bobine à double résonnance suivant l'une des revendications 1 à 9,
**caractérisée**
**en ce qu'**elle dispose de deux autres éléments de liaison, qui bloquent la bobine à double résonnance pendant l'émission par une autre bobine d'émission.

11. Bobine à double résonnance suivant la revendication précédente,
**caractérisée**
**en ce que** plusieurs bobines sont montées ensemble en un réseau.

12. Réseau de bobines à double résonnance suivant l'une des revendications 1 à 11.

13. Réseau suivant la revendication 12,
**caractérisé**
**en ce qu'**il possède un moyen de découplage de bobines individuelles à double résonnance.

14. Réseau suivant l'une des revendications 12 ou 13,
**caractérisé**
**en ce qu'**il possède un nombre de 2 à 128 bobines.

15. Réseau suivant l'une des revendications 12 à 14,
**caractérisé**
**en ce que** les bobines sont montées, de manière à entourer une tête au moins en partie.

16. Utilisation d'une bobine suivant l'une des revendications 1 à 11, d'un réseau suivant l'une des revendications 12 à 15, dans la spectroscopie par résonnance magnétique ou la tomodensitométrie par résonnance magnétique.
